# EUROPEAN PATENT APPLICATION

(11) **EP 1 849 459 A1**
(43) Date of publication of application: **31.10.2007**
(21) Application number: 07250908.6
(22) Date of filing: 06.03.2007
(51) Int. Cl.: A61K 9/14, A61K 31/397, A61K 31/70

(54) **Ezetimibe compositions**

(30) Priority: 06.03.2006 US 779880 P
(71) Applicant: TEVA PHARMACEUTICAL INDUSTRIES LTD., 49131 Petah Tiqva (IL)
(72) Inventor: Pal, Boaz, Tel Aviv (IL); Zalit, Ilan, Rosh-Ha-Ayin (IL)
(74) Representative: Gallagher, Kirk James

(57) **Abstract**

Provided are ezetimibe compositions with improved solubility and increased bioavailability, methods of their preparation, and method of treatment using the same. An ezetimibe composition may be prepared, for example, by co-milling ezetimibe with at least one hydrophilic excipient.

## Description

### CROSS REFERENCE TO RELATED APPLICATION

This application claims the benefit of U.S. Provisional Patent Application filed March 6, 2006, entitled "Ezetimibe Compositions," Serial No. 60/779,880, which is incorporated herein by reference in its entirety.

### FIELD OF THE INVENTION

The invention encompasses ezetimibe compositions with improved solubility and increased bioavailability, methods for their preparation, and methods for treatment using the same.

### BACKGROUND OF THE INVENTION

Ezetimibe, or 1-(4-fluorophenyl)-3(R)-[3-(4-fluorophenyl)-3(S)-hydroxypropyl]-4(S)-(4-hydroxyphenyl)-2-azetidinone, apparently has the following chemical structure:

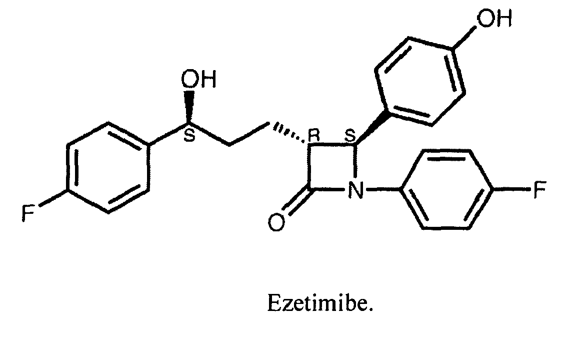

Ezetimibe is reported to be a white crystalline powder that is freely to very soluble in ethanol, methanol, and acetone, and practically insoluble in water. Ezetimibe is reported to have a melting point of about 163°C and to be stable at ambient temperature.

Ezetimibe is indicated mainly for primary hypercholesterolemia (administered alone or in combination with an HMG-CoA reductase inhibitor), homozygous familial hypercholesterolemia (administered with atorvastatin or simvastatin) and homozygous sitosterolemia [PDR prescribing information for Zetia®]. Ezetimibe is sold under the brand name Zetia®, which is marketed by Merck/Schering-Plough Pharmaceuticals. Zetia® is available as a tablet for oral administration containing supposedly 10 mg of ezetimibe. The inactive ingredients of Zetia are reported to be croscarmellose sodium, lactose monohydrate, magnesium stearate, microcrystalline cellulose, povidone, and sodium lauryl sulfate. The recommended dose is 10 mg once daily, administered with or without food according to the Zetia label.

Ezetimibe is reported to be practically insoluble in water. When a solid dosage form of ezetimibe is taken orally, the drug must dissolve in aqueous gastrointestinal fluids in, e.g., the patient's stomach before it can exert a therapeutic effect. A recurring problem with compressed solid oral dosage forms, such as tablets, capsules and caplets (i.e., capsule-shaped tablets) is that the rate of dissolution of the drug limits its biological availability.

Methods for improving dissolution by reducing particle size have been described in the past for water-insoluble drugs other than ezetimibe. However, particle size reduction is not always effective enough for increasing the dissolution rate of a drug to a certain required value. Many water-insoluble drugs have a strong tendency to agglomerate during the dosage form manufacturing process into larger particles with an overall decrease in effective surface area. Remington: The Science and Practice of Pharmacy, 20th ed. 656, 657 (A.R. Gennaro Ed., Lippincott Williams & Wilkins: Philadelphia 2000), incorporated by reference herein, contains a more thorough discussion of the concept of "effective surface area" and the effect of particle size on dissolution. A drug that has ostensibly been milled to a fine particle size will sometimes display dissolution characteristics of a larger particle due to agglomeration or similar effect.

There is a need in the art for ezetimibe compositions with improved solubility and increased bioavailability.

### SUMMARY OF THE INVENTION

The invention encompasses ezetimibe compositions with improved solubility and increased bioavailability, methods for their preparation, and method for treatment using the same.

In one aspect, the invention encompasses an ezetimibe composition comprising ezetimibe co-milled with at least one hydrophilic excipient. In another aspect, the invention encompasses a method for preparing an ezetimibe composition comprising co-milling ezetimibe and at least one hydrophilic excipient to form the ezetimibe composition.

The invention also encompasses ezetimibe compositions prepared by a method of the invention. The invention further encompasses a method for lowering cholesterol in a mammal in need thereof comprising administering a therapeutically effective amount of a composition of the invention.

### BRIEF DESCRIPTION OF THE FIGURES

Figure 1 illustrates dissolution profiles for milled and non-milled ezetimibe. This figure shows the effect of particle size and addition of starch on the dissolution rate.

Figure 2 illustrates dissolution profiles for compositions containing co-milled ezetimibe and starch compared to non-milled ezetimibe mixed with starch.

Figure 3 illustrates ezetimibe not milled with starch.

Figure 4 illustrates ezetimibe milled with starch.

### DETAILED DESCRIPTION OF THE INVENTION

The invention encompasses ezetimibe compositions with improved solubility and increased bioavailability, methods of their preparation, and methods of treatment using the same.

It has been discovered that the solubility of ezetimibe is increased by the addition of a hydrophilic excipient, such as a saccharide or polysaccharide, *e.g*., starch, to a composition containing milled ezetimibe.

In one aspect, the invention encompasses an ezetimibe composition comprising ezetimibe co-milled with at least one hydrophilic excipient. The hydrophilic excipient may include a saccharide or polysaccharide, for example starch, *e.g.* pregelatinized starch, mannitol, or sorbitol.

In a preferred embodiment, about 40% or more, preferably about 40% to about 70%, more preferably about 50% or more, and even more preferably about 60% or more, of the composition is dissolved in 40 minutes, and more preferably in 20 minutes or less. As used herein, percent dissolution is tested under conditions at least as stringent as dissolution in 450 ml phosphate buffer at pH 4.5 containing 0.15% sodium lauryl sulfate at 37°C using USP paddle method rotating at 50 RPM, preferably when measured by a UV detector at 248 nm.

Preferably, about 40% to about 70%, more preferably about 50% or more, and even more preferably of about 60% or more of the ezetimibe composition is dissolved in 20 minutes. Also preferably, about 50% or more of the ezetimibe composition is dissolved in 40 minutes.

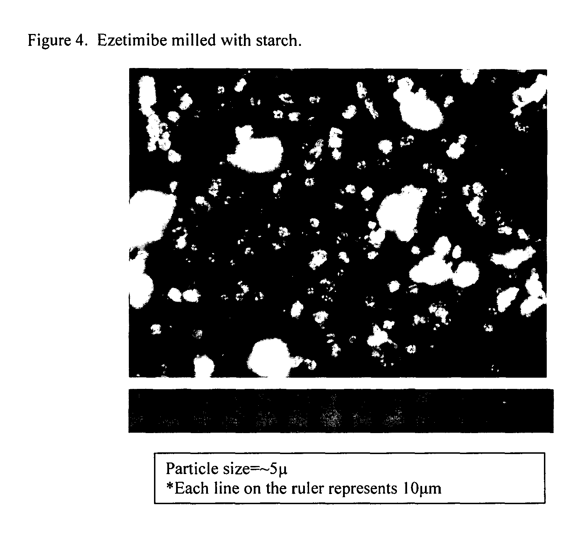

Co-milling can be carried out using conventional milling processes, which include jet milling, rolling milling, hammer milling, centrifugal-impact milling and sieving, pebble milling, cutter milling, or use of a mortar and pestle. The co-milled ezetimibe may have a particle distribution of d(0.5) less than or equal to about 25 µm, preferably less than or equal to 10 µm, and more preferably less than or equal to about 5 µm. The d(0.5) value can be estimated, for example, by microscopic observation, such as that exemplified in Figure 4.

The co-milled particles, *e.g*., ezetimibe, also may have a d (0.5) less than or equal to about 20 µm. Optionally, the co-milled particles have a d (0.9) less than or equal to about 20 µm. The nomenclature describing particle size is commonly and herein referred to as "d (0.9)"or "d(0.5)." For example, a d (0.9) of 20 µm, or d(0.9)=20 µm, means that 90% (by volume) of the particles have a size less than or equal to 20 microns; a d(0.5) of 5 µm, or d(0.5)=5 µm, means that 50% (by volume) of the particles have a size less than or equal to 5 microns, as tested by any conventionally accepted method such as the laser diffraction method. Accordingly, "d (0.9) less than or equal to about 20 µm" means a d(0.90) value of about 20 µm or less.

In one embodiment, the composition has an ezetimibe : hydrophilic excipient weight ratio of about 1:50 to about 50:1, preferably from about 1:10 to about 10:1, more preferably about 1:6 to about 1:3, and even more preferably about 1:5. The ezetimibe and hydrophilic excipient can be co-milled in the ratios described above.

In one embodiment, the ezetimibe composition is in the form of a granule. In another embodiment, a composition of the invention is a formulation further comprising one or more pharmaceutically acceptable excipient, in addition to the hydrophilic excipient. Preferably, about 40% or more of the ezetimibe composition comprising the additional pharmaceutically acceptable excipient is dissolved in 20 minutes. Also preferably, about 50% or more of the ezetimibe composition comprising the second pharmaceutically acceptable excipient is dissolved in 40 minutes. Optionally, the additional pharmaceutically acceptable excipient comprises at least one of a binder, filler or lubricant, more particularly povidone, microcrystalline cellulose, or magnesium stearate.

In another embodiment, the composition further comprises one or more dispersing agent, e.g., povidone or poloxamer.

In another aspect, the invention encompasses a method for preparing an ezetimibe composition comprising co-milling ezetimibe and at least one hydrophilic excipient to form the ezetimibe composition.

In one embodiment, the invention encompasses a method for preparing an ezetimibe composition comprising co-milling ezetimibe and at least one hydrophilic excipient, wherein about 40% or more, preferably about 40% to about 70%, more preferably about 50% or more, and even more preferably about 60% or more, of the ezetimibe composition is dissolved in 40 minutes, and preferably in 20 minutes or less. For example, about 40% to about 70%, preferably about 50% or more, and more preferably of about 60% or more of the ezetimibe composition is dissolved in 20 minutes. Optionally, about 50% or more of the ezetimibe composition is dissolved in 40 minutes.

An ezetimibe composition may be prepared by methods known in the art, such as dry granulation, wet granulation, blending, or direct compression. Preferably, the composition is prepared by wet granulation. The wet granulation mixture may contain a dispersing agent, which preferably comprises at least one of povidone or poloxamer. The co-milled ezetimibe and hydrophilic excipient may be combined with one or more pharmaceutically acceptable excipient, such as a binder, filler, or lubricant. For example, the co-milled ezetimibe and hydrophilic excipient may be combined with at least one of povidone, microcrystalline cellulose, or magnesium stearate. In one embodiment, the composition comprises about 50% by weight of pregelatinized starch, about 15% by weight of povidone, about 25% by weight of microcrystalline cellulose, and about 2% by weight of magnesium stearate.

The method for preparing the ezetimibe composition may further comprise slugging the co-milled ezetimibe and hydrophilic excipient, optionally after adding one or more pharmaceutically acceptable excipient, to form slugs, and milling the slugs into a powder; or passing the co-milled ezetimibe and hydrophilic excipient through a screen to form a granulate.

In another embodiment, the invention encompasses a method for preparing an ezetimibe composition comprising co-milling ezetimibe and starch to an average particle size of less than about 5 microns; pressing the ezetimibe and starch into slugs; milling the slugs; granulating the slugs with a granulation solution comprising ethanol and povidone to form granules; drying and sieving the granules through a 30-mesh screen; mixing the granules with microcrystalline cellulose and magnesium stearate; and compressing into tablet form, wherein about 40% or more, preferably about 40% to about 70%, more preferably about 50% or more, and even more preferably about 60% or more, of the ezetimibe composition is dissolved in 40 minutes, and preferably in 20 minutes or less.

Ezetimibe compositions of the present invention can contain inactive ingredients such as diluents, carriers, fillers, bulking agents, binders, disintegrants, disintegration inhibitors, absorption accelerators, wetting agents, lubricants, glidants, surface active agents, flavoring agents, and the like.

Diluents increase the bulk of a solid pharmaceutical composition and can make a pharmaceutical dosage form containing the composition easier for the patient and care giver to handle. Diluents for solid compositions include, for example, microcrystalline cellulose (e.g., Avicel^{®}), lactose, starch, pregelitinized starch, mannitol, polymethacrylates (e.g., Eudragit^{®}).

Carriers for use in the compositions may include, but are not limited to, lactose, starch, calcium carbonate crystalline cellulose, silicic acid, and the like.

Binders help bind the active ingredient and other excipients together after compression. Binders for solid pharmaceutical compositions include for example carbomer (e.g. carbopol), carboxymethylcellulose sodium, ethyl cellulose, hydroxypropyl cellulose (e.g. Klucel^{®}), hydroxypropyl methyl cellulose (e.g. Methocel^{®}) and povidone (e.g. Kollidon^{®}, Plasdone^{®})

Disintegrants can increase dissolution. Disintegrants include, for example, carboxymethylcellulose sodium (e.g. Ac-Di-Sol^{®}, Primellose^{®}), colloidal silicon dioxide, croscarmellose sodium, crospovidone (e.g. Kollidon^{®}, Polyplasdone^{®}), microcrystalline cellulose, and starch.

Adsorbing agents used include, but are not limited to, starch, lactose, kaolin, bentonite, colloidal silicic acid, and the like. Absorption accelerators may include, but are not limited to, quaternary ammonium base, sodium laurylsulfate, and the like. Wetting agents may include, but are not limited to, glycerin, starch, and the like.

A lubricant can be added to the composition to reduce adhesion and ease release of the product from a punch or dye during tableting. Lubricants include for example magnesium stearate, hydrogenated castor oil, mineral oil, polyethylene glycol, sodium stearyl fumarate, sodium lauryl sulfate.

Glidants can be added to improve the flowability of non-compacted solid composition and improve the accuracy of dosing. Excipients that can function as glidants include for example colloidal silicon dioxide, magnesium trisilicate, powdered cellulose, starch, talc.

Tablets can be further coated with commonly known coating materials. Capsules can be filled with powder or granule compositions of the invention.

The selection of excipients and the amounts to use can be readily determined by an experienced formulation scientist in view of standard procedures and reference works known in the art.

As described above, the ezetimibe formulations of the invention can be prepared by wet granulation. In wet granulation some or all of the active ingredients and excipients in powder form are blended and then further mixed in the presence of a liquid, typically water and/or alcohol, which causes the powders to clump up into granules. The granulation solution may contain a dispersing agent such as povidone and/or poloxamer. The granulate so formed is optionally screened and/or milled, dried and then screened and/or milled to the desired particle size. The granulate can then be tableted or other excipients can be added prior to tableting, such as a glidant and/or a lubricant. The granules can alternatively be filled into capsules, or sachets for example.

A preferred dosage form is a tablet. A tableting composition can be prepared conventionally by dry granulation. For instance, the active ingredient and excipients can be compacted into a "slug" or a sheet and then comminuted into compacted granules. The compacted granules can be compressed subsequently into a tablet.

As an alternative to dry granulation, a blended composition can be compressed directly into a compacted dosage form using direct compression techniques. Direct compression produces a tablet without granules. Excipients that are particularly well-suited to direct compression tableting include microcrystalline cellulose, spray dried lactose, dicalcium phosphate dihydrate and colloidal silica. The proper use of these and other excipients in direct compression tableting is known to those in the art with experience and skill in particular formulation challenges of direct compression tableting.

A capsule filling of the present invention can comprise any of the aforementioned blends and granulates that are described with reference to tableting; only they are not subjected to a final tableting step. When shaping the pharmaceutical composition into pill form, many commonly known excipient used in the art can be used.

The invention also encompasses ezetimibe compositions prepared by the methods of the invention. The invention also encompasses a method of lowering cholesterol in a mammal in need thereof by administering a therapeutically effective amount of the formulations of the invention. The amount of ezetimibe or pharmaceutically acceptable salt thereof contained in a composition of the invention for reducing cholesterol is not specifically restricted; however, the dose should be sufficient to treat, ameliorate, or reduce the condition.

The dosage of a pharmaceutical composition for reducing cholesterol according to the present invention will depend on the method of use, the age, sex, weight and condition of the patient. Typically, about 1 mg to 200 mg of ezetimibe may be contained in an administration unit form, preferably a 10 mg per tablet.

Ezetimibe may be present in an amount of about 1% to about 70% in compositions of the invention.

Having described the invention with reference to certain preferred embodiments, other embodiments will become apparent to one skilled in the art from consideration of the specification. The invention is further defined by reference to the following examples describing in detail the analysis of the crystals and processes for making the crystals of the invention. It will be apparent to those skilled in the art that many modifications, both to materials and methods, may be practiced without departing from the scope of the invention.

### EXAMPLES

### Example 1. Effect of particle size and addition of starch on dissolution rate of ezetimibe

Ezetimibe having particle distribution of d(0.5) 18µm and d(0.9) 66 µm was milled using a mortar and pestle. The ezetimibe was milled to an estimated size of d(0.5) 5µm and d(0.9) 20µm. The samples were prepared as follows:
1) 10 mg of milled ezetimibe.
2) 10 mg of non-milled ezetimibe.
3) 10 mg of milled ezetimibe blended with 50 mg of pregelatinized starch (e.g., starch 1500^{®}, available from Colorcon).
4) 10 mg of non-milled ezetimibe blended with 50 mg of starch 1500.
The dissolution profiles of each sample were tested using the following method:

| | |
|---|---|
| **Apparatus** | USP Apparatus 2 (Paddles) |
| **Medium** | 0.15% aqueous solution sodium lauryl sulfate and 6g/L NaH₂PO₄ pH=4.5 |
| **Volume** | 450 ml |
| **Temperature** | 37°C |
| **Speed** | 50 RPM |
| **Sampling points:** | 20, 40, 60, 80, 100, 120 and 140 minutes |

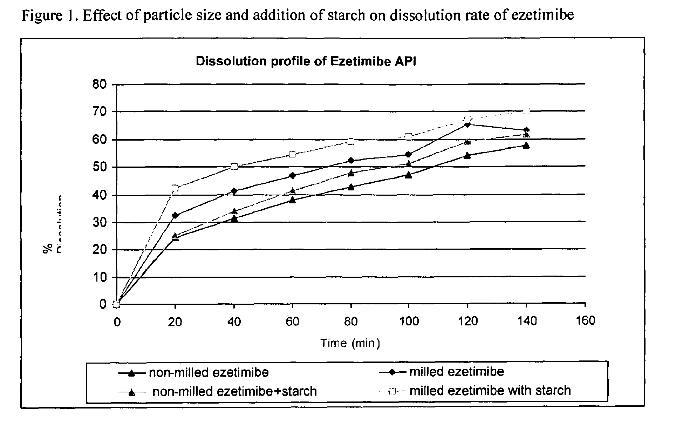

The samples analyzed on-line by a UV detector at a wavelength of 248 nm and the results are illustrated in Figure 1. As shown in Figure 1, the dissolution rate is increased when milled ezetimibe is blended with starch.

### Example 2: Formulations of Ezetimibe

Ezetimibe was first co-milled with starch in weight of ratio of 1:5. The mixture was then used to prepare ezetimibe tablets according the following procedure:

| **Ingredient** | **Amount (mg/dose)**** | **%** |
|---|---|---|
| ***Part I*** | | |
| Ezetimibe | 10.00 | 9.8% |
| Starch 1500 | 50.00 | 49% |

| ***Part II*** | | |
|---|---|---|
| *Granulation solution:* Povidone | 15.00 | 14.7% |
| Ethanol 97% * | 3.00 ml | - |

| ***Part III*** | | |
|---|---|---|
| Microcrystalline cellulose | 25.00 | 24.5% |

| ***Part IV*** | | |
|---|---|---|
| Magnesium stearate | 2.00 | 2.0% |
| Theoretical end weight | 102.0 | 100% |

| | | |
|---|---|---|
| * The granulation solvent is removed during the drying process ** The amounts refer to one tablet | | |

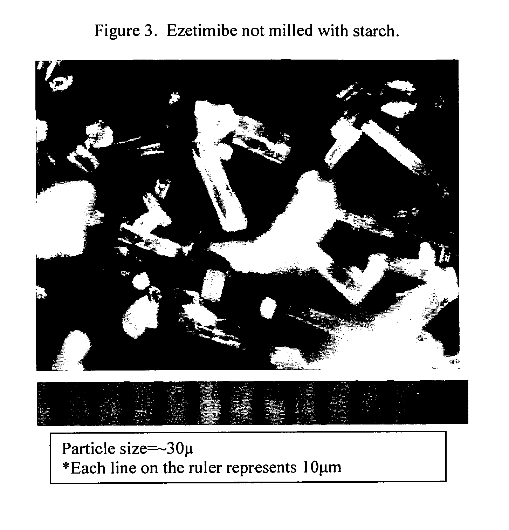

For the preparation of 150 tablets:
(a) 1.5 g of ezetimibe (having particle size distribution of d(0.5) 18µm and d(0.9) 66 µm) and 1.5 g of starch were milled with a mortar and pestle to a final ezetimibe particle size of less than about 5 microns as determined by microscope observation (see Figure 3: mixture before milling; Figure 4: mixture after milling). The remainder of the starch, 6g, was added in small amounts (approximately 500 mg in each addition) and milled with the ezetimibe after each addition.
(b) The mixture was pressed into slugs and then milled by a coffee grinder machine.
(c) Part II - Povidone was then dissolved in ethanol to obtain granulation solution.
(d) The milled slugs from step (b) were granulated with granulation solution from Part II. The granules were dried at 50°C under vacuum for 30 min. The dried granules were then sieved through a 30-mesh screen.
(e) The granules were then mixed with ingredients of Part III and Part IV.
(f) The final blend was then compressed into tablets.

The dissolution profiles of the following samples were tested:
i) Tablets prepared according to the above method.
ii) Granules prepared according to the above method.
iii) 10 mg of non-milled ezetimibe blended with 50 mg of starch.
iv) 10 mg of ezetimibe co milled with 50 mg of starch as described in step (a).

| | |
|---|---|
| Apparatus | USP Apparatus 2 (Paddles) |
| Medium | 0.15% aqueous solution sodium lauryl sulfate and 6g/L NaH₂PO_{4.} pH=4.5 |
| Volume | 450 ml |
| Temperature | 37°C |
| Speed | 50 RPM |
| Sampling points: | 20, 40, 60, 80, 100, 120 and 140 minutes |

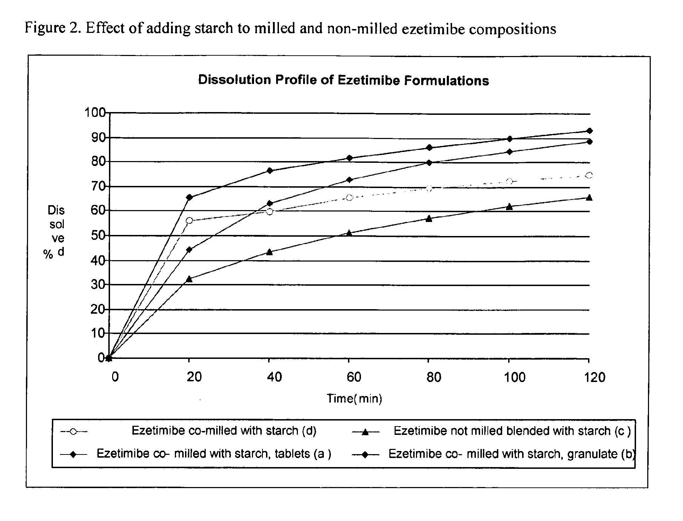

The samples were analyzed on-line by a UV detector at a wavelength of 248 nm. The results are illustrated in Figure 2. As shown in Figure 2, the dissolution rate was improved by co-milling with ezetimibe with starch in comparison with compositions where ezetimibe was milled alone.

## Claims

1. An ezetimibe composition comprising ezetimibe co-milled with at least one hydrophilic excipient.

2. The ezetimibe composition of any preceding claim, wherein the hydrophilic excipient comprises a saccharide or a polysaccharide.

3. The ezetimibe composition of any preceding claim, wherein the hydrophilic excipient comprises starch.

4. The ezetimibe composition of any preceding claim, wherein the hydrophilic excipient comprises pregelatinized starch.

5. The ezetimibe composition of any preceding claim, wherein the ezetimibe : hydrophilic excipient weight ratio is about 1:10 to about 10:1.

6. The ezetimibe composition of any preceding claim, wherein the ezetimibe : hydrophilic excipient weight ratio is about 1:6 to about 1:3.

7. The ezetimibe composition of any preceding claim, wherein the ezetimibe : hydrophilic excipient weight ratio is about 1:5.

8. The ezetimibe composition of any preceding claim, wherein the co-milled ezetimibe has a d(0.5) less than or equal to about 25 µm.

9. The ezetimibe composition of any preceding claim, wherein the co-milled ezetimibe has a d(0.5) less than or equal to about 10 µm.

10. The ezetimibe composition of any preceding claim, wherein the co-milled ezetimibe has a d(0.5) less than or equal to about 5 µm.

11. The ezetimibe composition of any preceding claim, wherein the co-milled ezetimibe has a d(0.9) less than or equal to about 20 µm.

12. The ezetimibe composition of any preceding claim, further comprising one or more of a pharmaceutically acceptable excipient selected from a binder, filler, or lubricant.

13. The ezetimibe composition of any preceding claim, further comprising one or more of a pharmaceutically acceptable excipient selected from povidone, microcrystalline cellulose, or magnesium stearate.

14. The ezetimibe composition of any preceding claim, further comprising a dispersing agent.

15. The ezetimibe composition of any preceding claim, further comprising at least one of povidone or poloxamer.

16. The ezetimibe composition of any preceding claim, in the form of a granule or granules.

17. The ezetimibe composition of any preceding claim, wherein about 40% or more of the composition is dissolved in 20 minutes in 450 ml of phosphate buffer at pH 4.5 containing 0.15% sodium lauryl sulfate at 37°C using a USP paddle method rotating at 50 RPM.

18. The ezetimibe composition of claim 17, wherein about 40% to about 70% of the ezetimibe composition is dissolved in 20 minutes.

19. The ezetimibe composition of any one of claims 17 or 18, wherein about 50% or more of the ezetimibe composition is dissolved in 20 minutes.

20. The ezetimibe composition of any one of claims 17-19, wherein about 60% or more of the ezetimibe composition is dissolved in 20 minutes.

21. The ezetimibe composition of any one of claims 1-16, wherein about 50% or more of the ezetimibe composition is dissolved in 40 minutes in 450 ml of phosphate buffer at pH 4.5 containing 0.15% sodium lauryl sulfate at 37°C using a USP paddle method rotating at 50 RPM.

22. A method for preparing an ezetimibe composition according to any preceding claim.

23. The method of claim 22, wherein the co-milling is performed using at least one of a jet mill, rolling mill, hammer mill, centrifugal-impact mill and sieve, pebble mill, cutter mill, or mortar and pestle.

24. The method of any one of claims 22-23 further comprising the steps of: (a) slugging the co-milled ezetimibe and hydrophilic excipient to form slugs; and milling the slugs into a powder, or (b) passing the co-milled ezetimibe and hydrophilic excipient through a screen to form a granulate.

25. The method of any one of claims 22-24 further comprising adding one or more pharmaceutically acceptable excipient to the co-milled ezetimibe and hydrophilic excipient; slugging the mixture to form slugs; and milling the slugs into a powder.

26. The method of any one of claims 22-25 further comprising granulating the co-milled ezetimibe and hydrophilic excipient.

27. The method of any one of claims 22-26 further comprising wet granulating the co-milled ezetimibe and hydrophilic excipient.

28. The method of any one of claims 22-27 further comprising combining the co-milled ezetimibe and hydrophilic excipient with a dispersing agent.

29. The method of any one of claims 22-28 further comprising combining the co-milled ezetimibe and hydrophilic excipient with at least one of povidone or poloxamer.

30. An ezetimibe composition prepared according to the process of any one of claims 22-47.

31. Use of an ezetimibe composition according to any one of claims 1-21 and 30 for the manufacture of a medicament for lowering cholesterol.

32. The ezetimibe composition of any one of claims 1-21 and 30 comprising about 50% by weight of pregelatinized starch, about 15% by weight of povidone, about 25% by weight of microcrystalline cellulose, and about 2% by weight of magnesium stearate.

33. The method of any one of claims 22-29 comprising the steps of:
(a) co-milling ezetimibe and starch to an average particle size of less than about 5 µm;
(b) pressing the co-milled ezetimibe and starch into slugs;
(c) milling the slugs;
(d) granulating the slugs with a granulation solution comprising ethanol and povidone to form granules;
(e) drying and sieving the granules through a 30-mesh screen;
(f) mixing the granules with microcrystalline cellulose and magnesium stearate; and
(g) compressing into tablet form.
